(19) Europäisches Patentamt
European Patent Office
Office européen des brevets

(11) Publication number: **0 404 255 A1**

# (12) EUROPEAN PATENT APPLICATION

(21) Application number: 90201588.2

(22) Date of filing: 19.06.90

(51) Int. Cl.5: C08G 69/16, C08G 69/44, C07D 227/087

(30) Priority: 22.06.89 NL 8901569

(43) Date of publication of application: 27.12.90 Bulletin 90/52

(84) Designated Contracting States: AT BE CH DE DK ES FR GB IT LI NL SE

(71) Applicant: STAMICARBON B.V.
Mijnweg 1
NL-6167 AC Geleen(NL)

(72) Inventor: Vriesema, Bindert Klaas
Burg. Luytenstraat 47
NL-6151 GE Sittard(NL)
Inventor: Van Geenen, Albert Arnold
Merkelbeekerstraat 82
NL-6441 KM Brunssum(NL)
Inventor: Lange, Ronald Frans Maria
Steenstraat 13
NL-6051 EP Maaasbracht(NL)
Inventor: Schröder, Christiaan
Rozenhoek 17
NL-6129 KK Stein (L.)(NL)

## (54) Acid halide and/or acyllactam functional compounds and polylactam block copolymers derived therefrom.

(57) The invention relates to acid halide and/or acyllactam functional compounds, to polylactam block copolymers derived therefrom by lactam polymerization at a lactam group, and to the use of such block copolymers in polyamide mixtures. The compounds contain liquid crystalline compounds or compounds which combine with acyllactam to form a compound with liquid crystalline properties. The products of this invention are useful as accelerators in the preparation of nylon block copolymers, in particular block copolymers with improved stiffness. The block copolymers are useful in products requiring good tensile strength, chemical resistance and/or heat resistance, and are particularly useful in electrical engineering applications and in the aircraft industry.

EP 0 404 255 A1

# ACID HALIDE AND/OR ACYLLACTAM FUNCTIONAL COMPOUNDS AND POLYLACTAM BLOCK COPOLYMERS DERIVED THEREFROM

## Background of the Invention

The invention relates to acid halide and/or acyllactam functional compounds, to polylactam block copolymers derived therefrom by lactam polymerization at a lactam group, and to the use of such block copolymers in polyamide mixtures.

Acid halide and acyllactam functional compounds are described, inter alia, in EP-A-67693 and EP-A-67694.

In these, acyllactam functional compounds are obtained by reacting a polyhydroxy compound with an acid halide and subsequently with lactam monomer. Examples of acid halides are carboxylic acid halide, phosphorylic acid halide or sulphonylic acid halide. The polyols applied in EP-A-67693 and EP-A-67694 preferably are polyether polyols, polyester polyols and polybutadiene polyols.

EP-A-67695 states that acid halides are highly suitable as activators or accelerators in the preparation of nylon block copolymers, in particular in the so-called RIM or RRIM systems, which require fast polymerization. Such nylon block copolymers contain blocks of a polyamide.

Polyamides are generally known polycondensates of mostly aliphatic dicarboxylic acids with 4-12 carbon atoms with mostly aliphatic diamines with 4-14 carbon atoms and/or of lactams with 6-12 carbon atoms. Examples of polyamides are: polyhexamethylene adipamide (nylon 6,6), polyhexamethylene azelamide (nylon 6,9), polyhexamethylene sebacamide (nylon 6,10), polyhexamethylene lauramide (nylon 6,12), polytetramethylene adipamide (nylon 4,6), polycaprolactam (nylon 6) and polylaurinolactam (nylon 12). The dicarboxylic acids and/or the diamines can also be aromatic.

Polyamides may also be composed of two or more dicarboxylic acids and/or two or more diamines, or of two or more lactams, and they may also consist of mixtures of two or more polyamides.

Polyamides have numerous applications. For certain of these it often is desirable to modify the properties of polyamides. Modification of properties is usually effected by synthesizing block copolymers consisting of one or more polyamide blocks and one or more blocks of another type of polymer. Thus, for instance, it is known that hydrophobic blocks in, for instance, polycaprolactam can reduce the sometimes undesired hydrophilic character of said substance. Thus, polylactampolyether block copolymers, for example, combine properties that render them very suitable for so-called engineering plastics.

## Summary of the Invention

Compounds have now been found of the general formula:

$P - [ - A-(-L-)_y - B]_x$ (I)

where:

A represents an oxygen atom or an -NH group,

B represents one of the following groups:

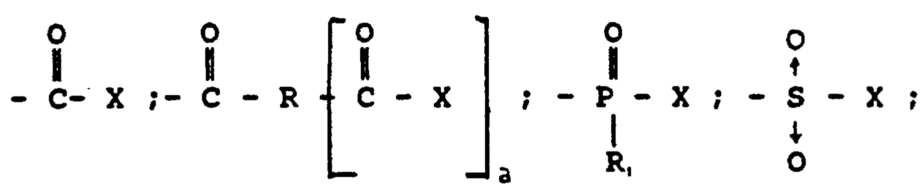

and where:

X is a halogen or (-L)

R represents an alkyl, aralkyl, alkaryl or aryl group, wherein alkyl, alkyl bound in aralkyl or alkaryl or is straight chained, branched or cyclic,

$R_1$ can be a monoalkyl, monoaryl, monoaralkyl, monoalkoxy, monoaryloxy, monoaralkyloxy or a halogen group, wherein alkyl, or alkyl bound in aralkyl or alkaryl is straight chained, branched or cyclic,

P represents a radical of a compound $P(AH)_x$, having a number average molecular weight of at least 150, with one or more OH or $NH_2$ groups, $P(AH)_x$ being a liquid crystalline compound or a compound with liquid

crystalline segments in the main and/or side chain, or a compound which combines with acyllactam to form a compound with liquid crystalline properties,
(-L-) represents an opened lactam ring,
(-L) represents an unopened lactam ring,
x is an integer with a value of 1 or higher,
y has a value of 0 or 1,
a is an integer with a value of 1 or higher.

It has also been found that the above-mentioned compounds can be polymerized to polyamide block copolymers by lactam polymerization at the terminal lactam group. Such compounds have the formula:

$P - [ - A - (-L-)_y - B - L - PL ]_x$

wherein A, B, P, -L-, x and y are defined as shown above and PL represents a polylactam block.

It has further been found that compounds of formula (I) are very suitable for use as accelerators in the preparation of nylon block copolymers, in particular block copolymers with improved stiffness. These block copolymers can be used for the same or similar applications as nylon 6.

The compounds of the above-mentioned general formula (I), in which X represents an unopened lactam ring, can be prepared per se by reacting a compound containing one or more -OH or $-NH_2$ groups, as defined, at elevated temperature of at least 80 °C, preferably between 80 °C and 250 °C, with an acyllactam in the presence of a catalyst, for example, an alkali metal lactamate, such as sodium or potassium lactamate, or a Lewis acid, such as magnesium stearate, magnesium chloride, zinc acetylacetonate. The temperature should not be so high as to give rise to the formation of decomposition products. In general it is more preferable to use temperatures between about 90° and and 250 °C. Such a conversion is carried out in molten lactam, in a solvent or dispersant, in which the polymer can be present in dissolved, partly dissolved or undissolved condition, or in the bulk, i.e., without solvent, dispersant or molten lactam. The molar ratio of catalyst and acyllactam may be any ratio which results in an acceptable rate of conversion and preferably ranges from 0.01 to 10, and more preferably 0.5 to 5.

The compounds of the above-mentioned general formulas (I), in which X represents a halogen, preferably Br or Cl, can be prepared by a similar technique, except that the first lactam eliminates the halogen. A tertiary amine is used to bind the halogen.

The molar ratio between acyllactam and OH or NH, groups of $P(AH)_x$ polymer may range from about 10 to 0.5, preferably from 5 to 1, the most preferred ratio being 1.

Acyllactam is here understood to mean reaction products of an acid chloride and a lactam, particularly caprolactam.

## Detailed Description of the Invention

Acyllactam is obtained according to the following reaction scheme:

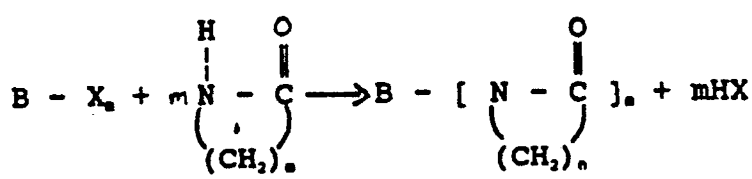

where B is defined above, n is an integer of 3-11, and preferably 5, X is a halogen, and m an integer higher than or equal to 2.

Liquid crystalline polymers that can be used in the subject invention possess at least one or more aromatic units, for instance:
units with an oxy- and a carboxyl-group;
units with two oxy-groups, and
units with two carboxyl-groups.

The formula of a unit with an oxy- and a carboxyl-group in general is

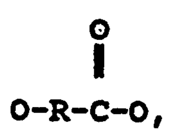

R containing at least an aromatic ring. Several non-limiting examples are:

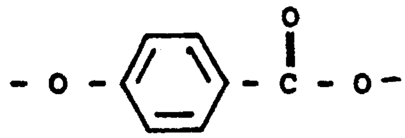
(IIa)

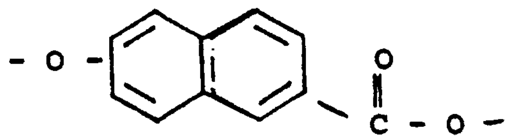
(IIb)

By preference the liquid crystalline polymer contains units of formula (IIa). These units originate from hydroxybenzoic acid or derivatives therefrom. Both the para and the meta substituted unit can be applied. By preference unit (IIa) is para substituted. In the aromatic ring one or more of the hydrogen atoms can be substituted by an alkyl or alkoxy group with one to four carbon atoms, by a halogen, for instance chlorine, bromine and/or fluorine, and/or by a phenyl group that may be substituted.

The formula of units with two oxy groups generally is O-R-O. Examples are:

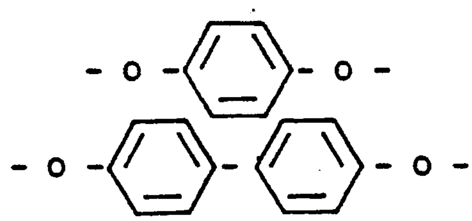
(IIIa)

(IIIb)

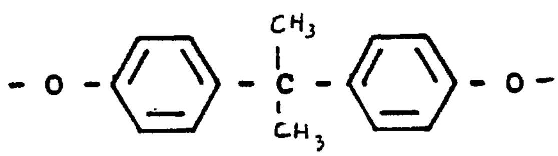
(IIIc)

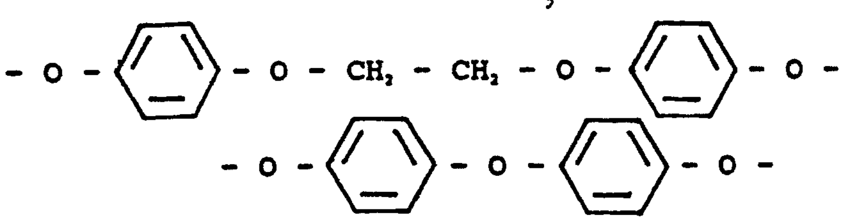
(IIId)

(IIIe)

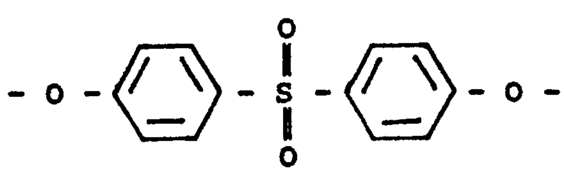
(IIIf)

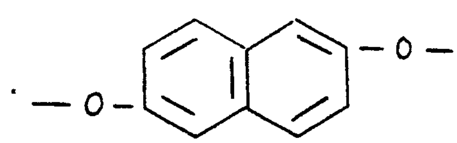
(IIIg)

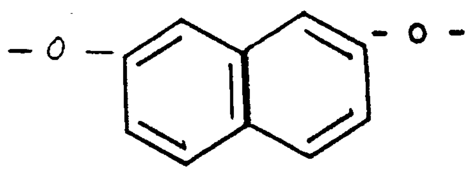

(IIIh)

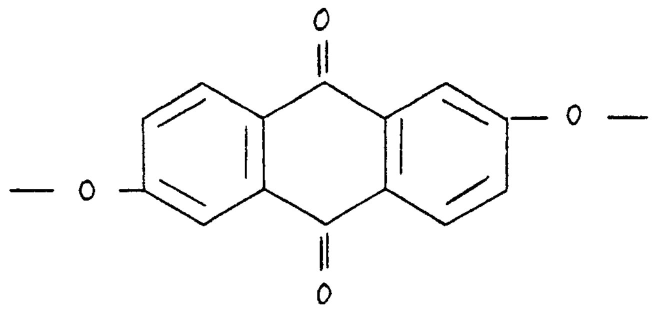

(IIIi)

The liquid crystalline polymer can also contain mixtures of the above-mentioned units, or dioxyalkanes, for instance ethylene glycol, neopentyl glycol, 1,4-butanediol. The prepolymer according to the invention preferably contains dioxyphenyl (IIIa) and/or biphenoxy (IIIb) groups, more particularly a biphenoxy group of formula (IIIb). The latter compound can be obtained from p,p′-biphenol. In the aromatic rings one or more hydrogen atoms may be substituted by an alkyl and/or alkoxy group having 1 to 6 carbon atoms, a halogen and/or a phenyl group, which itself may be substituted.

The aromatic units with two carboxyl groups can be obtained from aromatic dicarboxylic acids or from the corresponding esters, for instance terephthalic acid, isophthalic acid, 2,6-naphthalene dicarboxylic acid, 2,7-naphthalene dicarboxylic acid, dibenzoic acid, 4,4′-dicarboxydiphenyl sulphone, 4,4′-dicarboxydiphenyl ethane, 4,4′-dicarboxydiphenyl sulphide, 4,4′-dicarboxydiphenyl ether, 4,4′-dicarboxydiphenyl methane, 4,4′-dicarboxydiphenoxy ethane, 2,2-bis (4-carboxyphenyl) propane.

Preferably the aromatic units with two carboxy groups are obtained from terephthalic acid, and/or derivatives therefrom. In the aromatic ring one or more hydrogen atoms may be substituted by an alkyl and/or alkoxy group, a halogen and/or a phenyl group. The phenyl group may also be substituted. By preference the units with two carboxyl groups are obtained from non-substituted terephthalic acid and/or isophthalic acid.

Depending on the desired properties of the polymer composition, the liquid crystalline polymer may also contain an amide-containing unit, which can be obtained, inter alia, from known substances, for instance p-aminobenzoic acid, p-amino-phenol, p-N-methyl-aminophenol, p-phenylene diamine, N-methyl-p-phenylene diamine, N,N′-dimethyl-p-phenylene diamine, m-amino-phenol, 4-amino-1-naphthol, 4-amino-4′-hydroxy-diphenyl, 4-amino-4′-hydroxy-phenyl ether, 4-amino-4′-hydroxy-diphenyl methane, 4-amino-4′-hydroxy-diphenyl ethane, 4-amino-4′-hydroxy-diphenyl sulphone, 4-amino-4′-hydroxy-diphenyl sulphide, 4,4′-diamino-phenyl sulphide, 4,4′-diamino-diphenyl sulphone, 2,5-diamino-toluene, 4,4′-ethylene phenyldiamine, 4,4′-diamino-diphenoxy ethane, 4,4′-diamino-diphenyl methane, 4,4′-diamino-diphenyl ether.

Besides the above-mentioned units, the liquid crystalline part may also contain sulphon-, urethaner, carbonate-, imide-, ether-, keto-, urea-, sulphide-, azo-, anhydride-and thioester-groups.

By preference the liquid crystalline polymer is composed of units of the formula IIa, IIIb and of terephthalic acid and/or isophthalic acid.

Liquid crystalline polymers with the liquid crystalline segment in a side branch of the polymer can also be used in the subject invention. Such liquid crystalline polymers are described in A. Ciferri et al., Polymer Liquid Crystals, Academic press 1982, chapter 2, pp. 35-40.

As compounds with liquid crystalline segments in the main chain and/or side chain use can also be made of polymers such as polyols, polyester polyols, polybutadiene polyols or siloxane-containing polyols with liquid crystalline segments, such as for instance

HO - polyol - LCP - polyol - OH

or

HO - LCP - polyol - LCP - OH etc.

LCP is here understood to mean the liquid crystalline segment.

Use can also be made of LCP - polyols with liquid crystalline segments in the side chain.

Furthermore, use can be made of compounds obtained by the reaction of a polyol with a polymer with acyl halide terminal groups, the polymer being an LCP or a compound with LCP segments or a compound

with LCP segments in the side chain.

As polyols use can be made of compounds of the following general formula:

$R_2 - (OH)_x$

where x is an integer having a value of 2 or more, preferably 2 to 4.

The $R_2$ group in the formula $R_2$-(OH), may represent a hydrocarbon (preferably with a molecular weight of at least 28), a polyether or a polysiloxane group. Molecular weights of polymers or polymer segments in this context are understood to mean the number average molecular weights, which can be determined using known techniques such as gel-phase chromatography. The terms polysiloxane groups or polysiloxane segments relate to groups or segments that contain at least 50 weight per cent of one or more units of the following formula:

$$-(-\overset{\displaystyle R_3}{\underset{\displaystyle R_4}{\overset{|}{\underset{|}{Si}}}}-O-)-$$

where

$R_3$ represents alkyl, aryl, cycloalkyl, alkaryl and/or aralkyl, and

$R_4$ represents alkyl, aryl, cycloalkyl, alkaryl and/or aralkyl.

Polysiloxane groups or segments usually also contain other groups, for example ether groups with lower alkyls such as ethane or methane. Such ether groups often are terminal groups in the chain of repeating siloxane units. These ether groups may form up to 50 weight per cent of the polysiloxane group, but preferably they are present in an amount below 30 weight per cent. Preferably, however, $R_2$ is a hydrocarbon group or a polyether group. Examples of hydrocarbon groups are alkylene groups in the case of diols such as ethylene glycol, or polymeric or hydroxyl groups. A polyoxypropylene segment containing two or more hydroxyl groups is an example of a polyether group. Examples of compounds that contain hydroxyl groups and that can be used in the above-mentioned process are ethylene glycol, propylene glycol, poly(oxybutylene) glycol, poly(oxyethylene) glycol, poly(oxypropylene) diol, poly(oxypropylene)triol, poly(oxypropylene) tetrol, polybutadiene diol, polydimethyl siloxanes that contain hydroxyl groups, and combinations of such polydimethyl siloxanes, for instance block polymers of poly(oxypropylene) and poly-(oxyethylene) that contain hydroxyl groups.

Use can also be made of compounds of the general formula $P(AH)_x$, which, after conversion with acyllactam, a non-limiting example of which is terephthaloyl biscaprolactam, yield a compound of the general formula (I) with liquid crystalline properties.

The polyamines used according to the invention are polyamines with at least two groups. Preferably, they are chosen from the group consisting of polyoxyalkylene polyamines, polyalkadiene polyamines, polyalkene polyamines and combinations thereof.

The polyamine has an average molecular weight such that the polyamine provides an elastomer segment in the polyamide ultimately prepared, while the lactam polymerization provides the hard crystalline segment in the polyamide. Preferably the average molecular weight of the polyamine is between 300 and 10,000, more preferably at least 500, and an average molecular weight of at least 1000 is recommended. Elastomer segments contribute to a glass transition temperature Tg below 0 °C, preferably below -25 °C, as soon as they are incorporated in the nylon block copolymer. This glass transition temperature is determined by differential scanning calorimetry under nitrogen at a scanning rate of 10-20 °C per minute. The amount of elastomer segments in the nylon block copolymer may range from 10 to 90 weight per cent, depending on the properties ultimately desired.

Examples of suitable polymeric hydrocarbon polyamines are polybutadiene diamine, polybutadiene polyamines and butadiene acrylonitrile polyamines. Examples of suitable polyether polyamines are poly-(oxybutylene) diamine, poly(oxyethylene) diamine, poly(oxypropylene) diamine, poly(oxypropylene) triamine, poly (oxypropylene) tetramine and combinations thereof such as, for instance, block copolymers of poly-(oxypropylene) and poly(oxyethylene) with at least two functional amine groups. Polyether polyamines preferably used are poly(oxypropylene) triamines with an average molecular weight of at least 2000.

Acyllactams can be prepared from an acid halide and a lactam. Suitable acid halides for use in the framework of the present invention are acid halides as described in EP-A-67694. Examples of such acid halides are adipoyl chloride, terephthaloyl chloride, trimesoyl chloride, oxalyl chloride, isophthaloyl chloride, carbonyl chloride, phosphorus oxychloride, etc.

The acid chloride is reacted with lactam, by preference with caprolactam, in the presence of an acid-binding agent such as a tertiary amine or pyridine to yield an acyllactam.

Subsequently, the acyllactam is reacted, in the above-mentioned manner, with a polymer with OH or $NH_2$ groups as defined above. Compounds of the general formula (I) can also be prepared in a way known per se by reacting, at an elevated temperature of at least 80 °C, preferably between 90 °C and 250 °C, and in the presence of an acid-binding agent such as a tertiary amine or pyridine, a polymer containing one or more -OH or $-NH_2$ groups, as defined above, with an acid halide and subsequently with lactam. In the presence of a lactam polymerization catalyst the product of the general formula (I) can then be polymerized in molten lactam or in a suitable dispersant or in the bulk, yielding a polyamide block copolymer of the general formula

$P - [ - A - (-L-)_y - B - L - PL ]_x$

where the symbols A, B, P, - L -, x and y have the above-defined meanings and PL represents a polylactam block.

If x = 1 in the above formula of the compounds according to the subject invention, then so-called two-block or AB block copolymers of the general formula

$P - A - (-L-)_y - B - L - PL$

are obtained, where the symbols A, B, P, -L-, PL and y have the meanings defined above, which is very desirable for a block copolymer to be used as a compatibilizer. In the interface between two non-compatible polymers, such AB block copolymers take an orientation such that the blocks stick in the polymer components with which they are compatible, so that not only good stabilization but also strong bonding of the two components is obtained.

The lactam polymerization of an acyllactam compound of the formula shown above is carried out in a manner known per se in the presence of a basic lactam polymerization catalyst. Such catalysts and such polymerizations are known and are described, for instance, in EP-A-67693, EP-A-67694 and EP-A-67695. Some examples are: sodium lactamate, potassium lactamate and lactam magnesium bromide and chloride. A minor quantity of catalyst is already sufficient, for instance less than 1 mole % relative to the lactam to be polymerized, but larger quantities, for instance up to 3 mole %, can also be used.

The weight ratios between lactam monomer and acyllactam compound may vary widely. In general, they lie between 5 : 95 and 95 : 5, preferably between 30 : 70 and 70 : 30. As more lactam monomer is polymerized at the acyllactam compound the molecular weight of the polylactam block will increase.

The polylactam block should have a number average molecular weight of at least 1000, preferably at least 4000. In general the number average molecular weight will be at most 50,000. Higher molecular weights, though possible, do not offer advantages. The usual modifications of the lactam polymerization to polyamide, and so in this case to a polyamide block, may be applied.

The lactam used for the formation of the polyamide block in the subject block polymers is preferably the same lactam as used for the acyllactam. More in particular, caprolactam is used both for the acyllactam and for the formation of the polyamide block. However, addition of a minor quantity of pyrrolidone gives an accelerated reaction.

It may be essential to carry out the preparation of the polymer in the presence of one or more compounds which are normally used in polymers, such as fillers, plasticizers, flameproofing agents, stabilizers, impact modifiers and reinforcing fibres such as asbestos, glass fibres or carbon fibres, if characteristics such as increases stiffness, impact resistance, and fire resistance are desired.

Objects wholly or partially made of the polymer composition according to the invention possess excellent properties with respect to tensile strength, chemical resistance and heat resistance. This makes the polymer according to the invention very suitable for use in electrical engineering and in the aircraft industry.

The invention will be further elucidated by the following examples, without being restricted thereto.

## Example 1

### Preparation of liquid crystalline diacid chloride

A solution of 34.1 g terephthaloyl chloride and 17.8 g triethylamine in 500 ml diethyl ether was heated to 35 °C. While this solution was being stirred continuously, during a period of about 4 hours' time, 9.2 g hydroquinone, dissolved in 300 ml diethylether, was added dropwise. After complete addition and post-reacting for two hours at 35 °C, the mixture was cooled to 5 °C. All operations were carried out under a dry

nitrogen atmosphere. Triethylamine hydrochloride which was formed was washed out at this temperature by extracting the mixture 3 times with 150 ml water of 5 °C. After drying the magnesium sulphate the solvent was removed using a rotary film evaporator.

Example 2

Preparation of liquid crystalline acyllactam compound

50 g of the liquid crystalline diacid chloride obtained according to Example 1 was heated to 60 °C in 700 ml chloroform. Subsequently, with continuous stirring, a solution of 19.9 g caprolactam and 17.8 g triethylamine in 150 ml chloroform was added dropwise during a period of about one hour. After complete addition and post-reacting for 5 hours at 62 °C (reflux), the mixture was cooled to 20 °C. All operations were carried out under a dry nitrogen atmosphere. The triethylamine hydrochloride formed was subsequently removed by 3 extractions with 100 ml water each. After drying with magnesium sulphate the dispersant was removed using a rotary film evaporator.

Example 3

Preparation of acyllactam-terminated polyether-LCP-polyether compound

A solution of 2000 g PPG 2000 (polypropylene ether glycol with a molecular weight of 2000 g/mole) and 107 g triethylamine in 2000 ml toluene was heated at 100 °C. Then, at the same temperature, during a period of about 2 hours' time, a solution of 221 g of diacid chloride, obtained according to Example 1, in 2700 ml toluene was added dropwise. After complete addition and 15 hours' post-reacting, the mixture was cooled to 25 °C and the triethylamine hydrochloride was removed by filtration over a G3 glass filter. 357 g isophthaloyl biscaprolactam was added to the filtrate, following which the solvent (toluene) was evaporated at reduced pressure. The solvent-free mixture was subsequently reacted for 15 hours at 160 °C while being stirred, yielding the acyllactam-terminated polyether-LCP-polyether prepolymer.

Example 4

Preparation of acyllactam-terminated polyether-LCP compound

Acyllactam-terminated LCP-polyether-LCP compound was obtained by heating a mixture of 940 g Caradol (R) 36-3 (polyether triol with a molecular weight of 4700 g/mole; Shell), 358 g liquid crystalline acyllactam compound according to Example 2 and 0:6 g dry magnesium chloride (catalyst) for 5 hours at 150 °C while stirring continuously.

Example 5

Preparation of acyllactam-terminated polyester-LCP-polyester compound

A solution of 49.7 g p-hydroxybenzoic acid and 36.5 g terephthaloyl chloride in 600 ml tetrahydrofuran was heated to 60 °C. With continuous stirring, 40 g triethylamine, dissolved in 50 ml tetrahydrofuran, was added dropwise in about 20 minutes' time. After complete addition and 5 hours' post-reacting at 60 °C, the mixture was cooled to room temperature. All operations were carried out under a dry nitrogen atmosphere. The triethylamine hydrochloride was washed out with 1.5 l water to which 5 ml concentrated hydrochloric acid had been added. After filtration the mixture was washed with 1.5 1 10% sodium bicarbonate solution in order to remove any starting products present. After filtration the product was dried under vacuum at 70 °C for 18 hours.

20 g of the diacid chloride thus prepared was added, in solid form, at 190 °C in 15 minutes' time to a solution of 198.6 9 of a hydroxy functional polyester (composition: 57.7% terephthalic acid; 42.3% neopentylglycol) in 200 ml diphenyl ether. After addition of 150 ml diphenyl ether the reaction mixture was stirred for 18 hours at 190 °C. After cooling to 60-70 °C the mixture was filtered off and washed with, successively, 1.5 l toluene and 2 l methanol. After drying of the diol at 100 °C under vacuum, a mixture of the diol and 35.3 g isophthaloyl biscaprolactam in 600 ml toluene was stirred for 3 hours at 140 °C. After removal by distillation of the solvent, the desired prepolymer was obtained by stirring the mixture for 4 hours at 150 °C.

Example 6

Preparation of acyllactam functional polyether-LCP-polyether compound

A mixture of 2000 g Jeffamine (R) D2000 (polypropylene etherdiamine with a molecular weight of 2000 g/mole; Texaco) and 298 g liquid crystalline acyllactam compound, obtained according to Example 2, was heated at 150 °C during 10 hours while being stirred. The prepolymer thus formed was a good activator for the polymerization of caprolactam to nylon-6 block copolymer.

Example 7

Preparation of nylon block copolymer

58 g acyllactam functional prepolymer, obtained according to Example 4, was dissolved in 47 g dry caprolactam in an Erlenmeyer flask at 100 °C (solution A). In another Erlenmeyer flask 9 g caprolactam magnesium bromide (1 molar in caprolactam) was dissolved in 96 g caprolactam at 130 °C and subsequently cooled to 100 °C (solution B). The solutions A and B were mixed intensively, following which the mixture was poured into a 20x20x0.4 cm sheet mould. The mould temperature was 140 °C. After 20 minutes the mould was opened and a nylon sheet with an E-modulus of 2200 $N/mm^2$ was obtained.

## Claims

1. A compound of the formula:

$P - [ - A-(-L-)_y- B]_x$(I)

wherein:

A represents an oxygen atom or an -NH group,

B represents one of the following groups

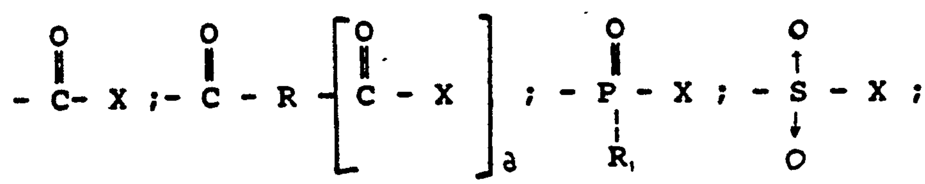

and wherein:

X is a halogen or (-L),

R represents an alkyl, aralkyl, alkaryl or aryl group, wherein alkyl, or alkyl bound in aralkyl or alkaryl is straight chained, branched or cyclic,

$R_1$ can be a monoalkyl, monoaryl, monoaralkyl, monoalkoxy, monoaryloxy, monoaralkyloxy or a halogen group, wherein alkyl, or alkyl bound in aralkyl or alkaryl is straight chained, branched or cyclic,

P represents a radical of a compound $P(AH)_x$, having a number average molecular weight of at least 150, with one or more OH or NH, groups, $P(AH)_x$being a liquid crystalline compound or a compound with liquid crystalline segments in at least one of a main and a side chain, or a compound which combines with

acyllactam to form a compound with liquid crystalline properties,
(-L-) represents an opened lactam ring,
(-L) represents an unopened lactam ring,
x is an integer with a value of 1 or higher,
y has a value of 0 or 1,
a is an integer with a value of 1 or higher.

2. A compound according to claim 1, wherein P comprises at least one member of the group consisting of:

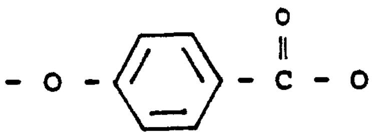
(IIa)

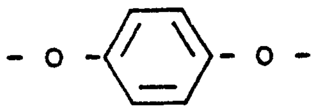
(IIIa)

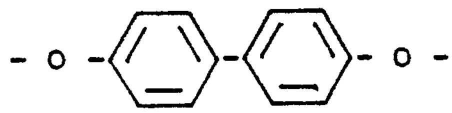
(IIIb)

3. A compound according to claim 1, wherein $P(AH)_x$ comprises at least one aromatic unit having an oxy group and a carboxyl group.

4. A compound according to claim 1, wherein P(AH). comprises at least one aromatic unit having two oxy groups.

5. A compound according to claim 1, wherein $P(AH)_x$comprises at least one aromatic unit having two carboxyl groups.

6. A polyamide block copolymer comprising at least one polyamide block and at least one other polymer block and having the formula:
$P - [ - A - (-L-)_y - B - L - PL ]_x$
wherein:
A represents an oxygen atom or an -NH group,
B represents one of the following groups

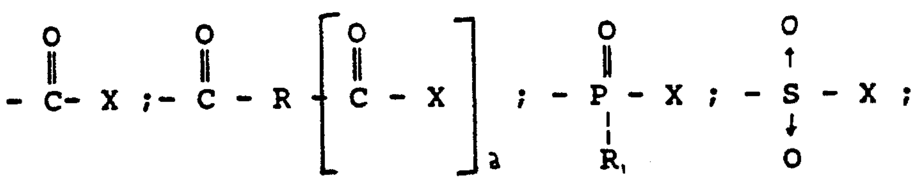

and wherein:
X is a halogen or (-L)
R represents an alkyl, aralkyl, alkaryl or aryl group, wherein alkyl, or alkyl bound in aralkyl or alkaryl is straight chained, branched or cyclic,
$R_1$ can be a monoalkyl, monoaryl, monoaralkyl, monoalkoxy, monoaryloxy, monoaralkyloxy or a halogen group, wherein alkyl, or alkyl bound in aralkyl or alkaryl is straight chained, branched or cyclic,
P represents a radical of a compound $P(AH)_x$, having a number average molecular weight of at least 150, with one or more OH or $NH_2$ groups, $P(AH)_x$ being a liquid crystalline compound or a compound with liquid crystalline segments in at least one of a main and a side chain, or a compound which combines with acyllactam to form a compound with liquid crystalline properties,
(-L-) represents an opened lactam ring,
(-L) represents an unopened lactam ring,
x is an integer with a value of 1 or higher,
y has a value of 0 or 1, and
PL represents a polylactam block.

7. A polyamide block copolymer according to claim 6, wherein x = 1.

8. Process for the preparation of acyllactam compounds of the general formula

P - [- A - (-L-)$_y$- B]$_x$

wherein:

A represents an oxygen atom or an -NH group,

B represents one of the following groups

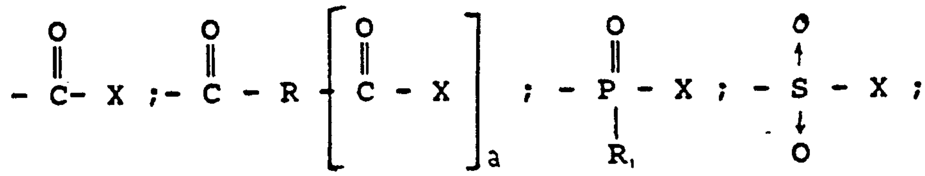

and wherein:

X is a halogen or (-L)

R represents an alkyl, aralkyl, alkaryl or aryl group, wherein alkyl, or alkyl bound in aralkyl or alkaryl is straight chained, branched or cyclic,

$R_1$ can be a monoalkyl, monoaryl, monoaralkyl, monoalkoxy, monoaryloxy, monoaralkyloxy or a halogen group, wherein alkyl, or alkyl bound in aralkyl or alkaryl is straight chained, branched or cyclic,

P represents a radical of a compound $P(AH)_x$, having a number average molecular weight of at least 150, with one or more OH or $NH_2$ groups, $P(AH)_x$ being a liquid crystalline compound or a compound with liquid crystalline segments in at least one of a main and a side chain, or a compound which combines with acyllactam to form a compound with liquid crystalline properties,

(-L-) represents an opened lactam ring,

(-L) represents an unopened lactam ring,

x is an integer with a value of 1 or higher,

y has a value of 0 or 1,

comprising reacting a compound $P(AH)_x$ containing one or more -OH or -NH, groups at a temperature of at least 80 °C with an acyllactam, wherein $P(AH)_x$ is a liquid crystalline compound, a compound with liquid crystalline segments in at least one of the main chain and the side chain or a compound which combines with acyllactam to form a compound with liquid crystalline properties.

9. A process according to claim 4, wherein the $P(AH)_x$ and acyllactam are reacted in the presence of a catalyst.

10. A process according to claim 4, wherein the reaction temperature is at least 90 °C and no more than 250 °C.

11. A process according to claim 7, wherein the ratio of catalyst to acyllactam is in the range of 0.01 to 10.

European Patent Office

# EUROPEAN SEARCH REPORT

Application Number

EP 90 20 1588

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 067 693 (MONSANTO)<br>--- | | C 08 G 69/16<br>C 08 G 69/44<br>C 07 D 227/087 |
| D,A | EP-A-0 067 694 (MONSANTO)<br>--- | | |
| D,A | EP-A-0 067 695 (MONSANTO)<br>----- | | |
| | | | **TECHNICAL FIELDS SEARCHED (Int. Cl.5)** |
| | | | C 08 G<br>C 07 D |

The present search report has been drawn up for all claims

| Place of search | Date of completion of the search | Examiner |
|---|---|---|
| THE HAGUE | 26-09-1990 | LEROY ALAIN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)